# EUROPEAN PATENT APPLICATION

(11) **EP 1 252 895 A1**
(43) Date of publication of application: **30.10.2002**
(21) Application number: 01948940.0
(22) Date of filing: 30.01.2001
(51) Int. Cl.: A61K 45/00, A61K 39/395, A61K 38/18, A61P 27/02, A61P 27/06, A61P 9/10

(54) **REMEDIES FOR OPHTHALMIC DISEASES**

(30) Priority: 31.01.2000 JP 2000021446; 05.09.2000 JP 2000268829
(71) Applicant: SANTEN PHARMACEUTICAL CO., LTD., Higashiyodogawa-ku, Osaka-shi, Osaka 533-8651 (JP)
(72) Inventor: HARA, Hideaki SANTEN PHARMACEU. CO., LTD., Ikoma-shi Nara 630-0101 (JP); YONEDA, Shinji SANTEN PHARMACEUTICAL CO., Ltd., Ikoma-shi Nara 630-0101 (JP); MIYAWAKI, Nobuaki SANTEN PHARMACEUTICAL CO., Ltd., Ikoma-shi Nara 630-0101 (JP); TANIHARA, Hidenobu, Tenri-shi, Nara 632-0093 (JP)
(74) Representative: Peaucelle, Chantal
(86) International application number: JP0100617
(87) International publication number: WO01056606

(57) **Abstract**

The present invention provides therapeutic agents for ophthalmopathy such as glaucoma or retinal diseases. Compounds having inhibitory activities of interleukin-1 such as intrinsic interleukin-1 receptor antagonist protein and an interleukin-1 neutralizing antibody have remarkable effects of inhibiting a decrease in cell number of a retinal ganglion cell layer, effects of inhibiting thinning of an inner plexiform layer and effects of lowering intraocular pressure.

## Description

### Technical Field

The present invention relates to protectants for optic nerve cells and therapeutic agents for ophthalmopathy comprising compounds having inhibitory activities of interleukin-1 as active ingredients.

### Background Art

A retina has a function to accept light from the outside and plays an important role for a visual function. The retina is structurally a tissue having thickness of 0.1 to 0.5 mm and consisting of ten layers such as a retinal pigment epithelial layer, an inner plexiform layer, a ganglion cell layer and a nerve fiber layer. Amacrine cells, which are nerve cells coupling with ganglion cell neurites to form synapses, exist in the inner plexiform layer. Since these cells respond well when irradiation of light is started and finished, the cells are considered to act as a detector for light intensity. Nerve cells having soma at the most inside site of the retina exist in the ganglion cell layer and closely participate in movement vision, peripheral vision, color vision, stereoscopic vision and the like. Retinal vessels, which are branches of central retinal artery and vein, run in the nerve fiber layer and supply oxygen and nutrition to an optic nerve.

Recently, so-called "neuroprotection" has been being established as an ultimate treatment of glaucoma (Ophthalmology, 40, 251-273, 1998). Since disorders of retinal blood circulation and optic nerve axonal transport finally cause dropout of nerve fibers due to ganglion cell death and progress to visual field defects in glaucoma, treatment to prevent or minimize the ganglion cell death leads to ultimate treatment of glaucoma. Actually, it was reported that disorders of a retinal ganglion cell layer and an optic disc were observed in rats treated with high intraocular pressure-induced ischemia (Graefes Arch. Clin. Exp. Ophthalmol., 234, 445-451, 1996), and a significant decrease in retinal ganglion cell density and a significant increase in glial cell density were observed after ten days of intraocular hypertension and a correlation was found between retinal ganglion cell loss and cell size in rabbits treated with methyl cellulose-induced intraocular hypertension (Graefes Arch. Clin. Exp. Ophthalmol., 234, S209-S213, 1996).

When the retinal vessel is occluded or constricted because of twitch, thrombus, arteriosclerosis or the like, the retinal blood circulation is disordered, and supply of oxygen and nutrition to the retina and the optic nerve is stopped. The retinal blood circulation disorder occupies a particularly important position in retinal diseases. Typical examples of symptoms accompanying the retinal blood circulation disorder are occlusion of retinal vessels wherein a retinal vein or a retinal artery is occluded or constricted, diabetic retinopathy, which contributes retinal detachment, and ischemic optic neuropathy exhibiting disturbance of visual function. Further, oxygen and nutrition are not supplied sufficiently because of the retinal blood circulation disorder, and retinal ganglion cells lead to death. The ganglion cell death is considered to participate in pathogenesis closely in other retinal diseases such as macular degeneration, retinitis pigmentosa and Leber's disease, too.

It is being clarified that apoptosis, which is one form of programmed cell death, participates in various aspects in ophthalmopathy. For example, it was reported that apoptosis occurs in retinal ganglion cells in ischemia/reperfusion damage in retina (J. Ocul. Pharmacol. Ther., 11, 253-259, 1995), retinal detachment (Arc. Ophthalmol., 113, 880-886, 1995), retinitis pigmentosa (Proc. Natl. Acad. Sci. USA, 91, 974-978, 1994, Invest. Ophthalmol. Vis. Sci., 35, 2693-2699, 1994), light-induced retinopathy (Invest. Ophthalmol. Vis. Sci., 37, 775-782, 1996), glaucoma (Invest. Ophthalmol. Vis. Sci., 36, 774-786,1995, Exp. Eye Res., 61, 33-44, 1995) and the like. Namely, though there are various causes, it is probable that the resulting disturbance of visual function is attributed to apoptosis of nerve cells constituting a visual information network.

On the other hand, with respect to inhibitors of interleukin-1 (IL-1), it was reported that when mouse arthritis models are treated with intrinsic IL-1 receptor antagonist protein (IL-1 ra) or an interleukin-1 neutralizing antibody, an inhibitory effect of inflammation is observed (Arthritis Rheum., 39, 797-809, 1996). Further, it was reported that IL-1 ra (Brain Res. Bull., 29, 243-246, 1992) or an IL-1 neutralizing antibody (Stroke, 26, 676-681, 1995) intraventicularly administered inhibits ischemic brain damage in rat cerebral ischemia models. WO 97/41844 discloses that an inhibitor of angiogenesis containing a combination of IL-1 ra and a tumor necrosis factor (TNF) antagonist inhibits appearance of pathological neovascularization. WO 96/09323 discloses that IL-1 ra has an inhibitory effect of inflammation and an immunosuppressive effect. Further, Japanese Laid-open Patent Publication No. 320072/1993 discloses that a zinc (II)-protoporphyrin IX complex having an inhibitory effect of IL-1 or an IL-1 neutralizing antibody reduces ischemic cerebral edema.

As mentioned above, it is known that inhibition of an action of interleukin-1 leads to various useful therapeutic effects.

However, it has not been reported how the inhibition of the action of interleukin-1 affects ophthalmopathy such as glaucoma or retinal diseases exemplified by occlusion of retinal vessels, diabetic retinopathy, ischemic optic neuropathy, macular degeneration, retinitis pigmentosa, Leber's disease and the like. It is also unknown how the inhibition affects ophthalmic tissues, particularly optic nerve cells such as retinal ganglion cells.

It is a very interesting subject to clarify a relation between interleukin-1 and the ophthalmic tissues, particularly the optic nerve cells and to study which therapeutic effect the inhibition of the action of interleukin-1 exhibits, thereby developing useful drugs.

### Disclosure of the Invention

Studying precisely by using various animal models, the present inventors found that optic nerve cells can be protected by inhibiting an action of interleukin-1. Namely, it turned out that the optic nerve cells can be protected by inhibiting the action of interleukin-1 since interleukin-1 damages the optic nerve cells. Specifically, the present inventors found that the optic nerve cells can be protected by administering intrinsic interleukin-1 receptor antagonist protein (IL-1 ra), which acts on an interleukin-1 receptor as an antagonist to interleukin-1, or an interleukin-1 neutralizing antibody, which acts on interleukin-1 directly and inhibits its action. Inhibition of a decrease in cell number of a retinal ganglion cell layer and inhibition of thinning of an inner plexiform layer were observed in experiments supporting these findings. Further, an excellent effect of lowering intraocular pressure was also observed. The above-mentioned findings show that compounds having inhibitory activities of interleukin-1 have effects of protecting the optic nerve cells and are useful as preventive agents or therapeutic agents for ophthalmopathy such as retinal diseases or glaucoma. Examples of the ophthalmopathy such as the retinal diseases or glaucoma are occlusion of retinal vessels, diabetic retinopathy, ischemic optic neuropathy, macular degeneration, retinitis pigmentosa, Leber's disease and the like.

The present invention provides protectants for the optic nerve cells such as retinal ganglion cells comprising the compounds having the inhibitory activities of interleukin-1 (IL-1) as active ingredients, more specifically the therapeutic agents for the ophthalmopathy such as the retinal diseases or glaucoma. Examples of the compounds having the inhibitory activities of interleukin-1 are compounds having antagonism to IL-1 (for example, intrinsic IL-1 receptor antagonist protein such as anakinra, intrinsic IL-1 receptor analogues such as a soluble IL-1 receptor and IL-1 receptor peptide, intrinsic IL-1 analogues such as IL-1 variants and peptide originating from IL-1, suramin and the like) and compounds which inhibit an activity of IL-1 (for example, an IL-1 neutralizing antibody, transforming growth factor (TGF)-β, α -melanocyte-stimulating hormone, glucocorticoid such as dexamethasone or prednisolone, a protoporphyrin IX zinc (II) complex and the like. The compounds also include compounds which inhibit production or release of IL-1 (for example, inhibitors of lipoxygenase such as nordihydroguaiaretic acid and n-propyl gallate, inhibitors of phosphodiesterase such as roliplam and the like). (See Nature, 343, 336-340, 1989; "Cytokine" [edited by Shinpei Kasakura, Nippon Igakukan, 1997]; Neurosci. Lett., 142, 45-47, 1992.)

With respect to effects of the present compounds having the inhibitory activities of interleukin-1 on the optic nerve cells, details will be described in the later section of "Pharmacological Tests". Studying effects on a hydraulic pressure-induced ischemic retinal disorder and effects on an N-methyl-D-aspartate (NMDA)-induced retinal disorder, remarkable effects of inhibiting the decrease in cell number of the retinal ganglion cell layer and effects of inhibiting the thinning of the inner plexiform layer were observed. Studying effects on intraocular pressure, the excellent effects of lowering intraocular pressure were confirmed.

The present compound having the inhibitory activity of interleukin-1 can be administered parenterally or orally. Examples of dosage forms of parenteral administration are eyedrops, injections, nasal drops and the like. Examples of dosage forms of oral administration are tablets, capsules, subtilized granules, granules, powders and the like. These can be prepared in conventional manners. For example, eyedrops can be prepared by optionally using an isotonic agent such as sodium chloride or concentrated glycerin; a buffer such as sodium phosphate or sodium acetate; a surfactant such as polyoxyethylenesorbitan monooleate, polyoxyl 40 stearate or polyoxyethylene hydrogenated castor oil; a stabilizer such as sodium citrate or disodium edetate; a preservative such as benzalkonium chloride or paraben; or the like; pH can be in a range acceptable for ophthalmic preparations, and it is preferably in a range of 4 to 8.

The dosage can be selected suitably depending on symptoms, age, dosage form and the like. In the case of eyedrops, they can be instilled once to several times per day with a concentration of 0.01 to 10% (w/v) solution. In the case of injections, the usual daily dosage is 0.0001 to 1 mg, which can be given in a single dose or several divided doses. In the case of oral preparations, the usual daily dosage is 10 µg to 1 g, which can be given in a single dose or several divided doses.

Results of pharmacological tests are shown below. These examples do not limit the scope of the invention, but are intended to make the invention more clearly understandable.

### Best Mode for Carrying out the Invention

### Pharmacological Tests

In order to study utility of compounds having inhibitory activities of interleukin-1 (IL-1), intrinsic IL-1 receptor antagonist protein (IL-1 ra, produced by R&D Systems Co., Ltd.) was tested for (1) an effect on a hydraulic pressure-induced ischemic retinal disorder, (3) an effect on an N-methyl-D-aspartate (NMDA)-induced retinal disorder and (4) an effect on normal intraocular pressure. An IL-1 neutralizing antibody (produced by R&D Systems Co., Ltd.) was tested for (2) an effect on the hydraulic pressure-induced ischemic retinal disorder.

### (1) Effect of IL-1 ra on hydraulic pressure-induced ischemic retinal disorder

Rats were anesthetized with 3% halothane and the anesthetized state was maintained with 1% halothane (halothane was vaporized with oxygen (0.5 liter/min) and a laughing gas (1.5 liters/min)). Next, mydriasis was caused in each right eye with an atropine ophthalmic solution, and a 30 G needle connected through a tube to an ophthalmic solution vessel of physiological saline hung from the ceiling was inserted into each anterior chamber to load it with hydraulic pressure of 130 mmHg to induce ischemia. After 45 minutes, the needle was taken out, and a retinal bloodstream was reperfused. Five minutes before the ischemic loading, 10 mM phosphate-buffered saline (solvent) and IL-1 ra (10 ng/eye) dissolved in the solvent were administered respectively to each vitreous body. Seven days after the administration, each eyeball was enucleated, then fixed in 2% paraformaldehyde-2.5% glutaraldehyde overnight, embedded with paraffin and sliced to prepare a histopathologic section (thickness: 3 µm) stained with hematoxylin-eosin (HE). A group to which the solvent was administered before the ischemia and a group to which IL-1 ra dissolved in the solvent was administered before the ischemia were referred to as "solvent administration group" and "IL-1 ra administration group" respectively. Three sections were chosen at random from eight sections containing an optic disc site therein prepared at intervals of 60 µm per eye. With respect to the chosen sections, photographs of the retina on a right or left side of 1 to 1.5 mm from the optic disc were taken to measure a cell number in a ganglion cell layer (GCL) and thickness of an inner plexiform layer (IPL). These results were compared with the corresponding measured values of "normal (untreated) group" which was not treated at all. Experimental results are shown in Table 1. Values in the table are the average.

**Table 1**

| Drug (Sample number) | Cell number in GCL (Number/mm) | Thickness of IPL (µm) |
|---|---|---|
| Normal (untreated) group (8) | 55 | 51 |
| Solvent administration group (7) | 30 | 28 |
| IL-1 ra administration group (8) (Dosage: 10 ng/eye) | 42 | 34 |

### (2) Effect of IL-1 neutralizing antibody on hydraulic pressure-induced ischemic retinal disorder

Ischemic loading was carried out for rats under 1% halothane anesthesia in the same manner as in the above (1) for 45 minutes. Five minutes before the ischemic loading, 10 mM phosphate-buffered saline (solvent) and an IL-1 neutralizing antibody (500 ng/eye) dissolved in the solvent were administered respectively to each vitreous body, and seven days after the administration, each eyeball was enucleated. Next, the same operations as in the above (1) were carried out, and a cell number in a ganglion cell layer (GCL) and thickness of an inner plexiform layer (IPL) were measured. A group to which the solvent was administered before the ischemia and a group to which the IL-1 neutralizing antibody dissolved in the solvent was administered before the ischemia were referred to as "solvent administration group" and "IL-1 neutralizing antibody administration group" respectively. These results were compared with the corresponding measured values of "normal (untreated) group" which was not treated at all. Experimental results are shown in Table 2. Values in the table are the average.

**Table 2**

| Drug (Sample number) | Cell number in GCL (Number/mm) | Thickness of IPL (µm) |
|---|---|---|
| Normal (untreated) group (20) | 59 | 45 |
| Solvent administration group (17) | 34 | 24 |
| IL-1 neutralizing antibody administration group (17) (Dosage: 500 ng/eye) | 39 | 29 |

### (3) Effect of IL-1 ra on NMDA-induced retinal disorder

A solution prepared by adding NMDA (dosage: 20 nmol/eye) to 10 mM phosphate-buffered saline was administered as a control to rats under 1% halothane anesthesia. A solution prepared by adding IL-1 ra (10 ng/eye) and NMDA (dosage: 20 nmol/eye) to 10 mM phosphate-buffered saline was administered as a test drug to rats under 1% halothane anesthesia. Seven days after the administration, each eyeball was enucleated, then fixed in 2% paraformaldehyde-2.5% glutaraldehyde overnight, embedded with paraffin and sliced to prepare a histopathologic section (thickness: 3 µm) stained with hematoxylin-eosin (HE). Next, the same operations as in the above (1) were carried out, and a cell number in a ganglion cell layer (GCL) and thickness of an inner plexiform layer were measured. A group to which the solvent and NMDA were administered and a group to which IL-1 ra and NMDA were administered were referred to as "[solvent+NMDA] administration group" and "[IL-1 ra+NMDA] administration group" respectively. These results were compared with the corresponding measured values of "solvent administration group" to which the solvent alone was administered. Experimental results are shown in Table 3. Values in the table are the average.

**Table 3**

| Drug (Sample number) | Cell number in GCL (Number/mm) | Thickness of IPL (µm) |
|---|---|---|
| Solvent administration group (13) | 56 | 46 |
| [Solvent+NMDA] administration group (14) | 25 | 20 |
| [IL-1 ra+NMDA] administration group (22) (Dosage: 10 ng/eye) | 42 | 27 |

### (4) Effect of IL-1 ra on normal intraocular pressure

IL-1 ra was dissolved in physiological saline (solvent) to prepare a 10 µg/ml test drug. A 0.4% oxybuprocaine hydrochloride ophthalmic solution was instilled drop by drop into both eyes of male Japanese white rabbits to anesthetize the rabbits locally, and then intraocular pressure was measured with an applanation tonometer. Next, the test drug (20 µl) was administered to an anterior chamber of one eye with a syringe provided with a 30 G needle. Four and six hours after administering the test drug, the 0.4% oxybuprocaine hydrochloride ophthalmic solution was instilled drop by drop into each administered eye to anesthetize the rabbits locally, and then intraocular pressure was measured. "Solvent administration group" was compared with a group to which IL-1 ra dissolved in the solvent was administered, i.e., "IL-1 ra administration group". Results are shown in Table 4. Values in the table are the average of a change in intraocular pressure based on a measured value (0) before the administration.

**Table 4**

| Drug (Sample number) | Change in intraocular pressure (mmHg) | | |
|---|---|---|---|
| | After 2 hr | After 4 hr | After 6 hr |
| Solvent administration group (3) | - 2.7 | - 1.7 | 1.0 |
| IL-1 ra administration group (3) (Dosage: 200 ng/eye) | -4.0 | -4.7 | -4.0 |

Tables 1 to 3 show that the decrease in cell number in the ganglion cell layer and the thinning of the inner plexiform layer are inhibited remarkably by administering the intrinsic interleukin-1 receptor antagonist protein (IL-1 ra) or the interleukin-1 neutralizing antibody to the vitreous body. Table 4 explicitly shows that IL-1 ra has the excellent effect of lowering intraocular pressure. These results of the pharmacological tests show that therapeutic agents for ophthalmopathy comprising the compounds having the inhibitory activities of interleukin-1 as active ingredients are useful as protectants for optic nerve cells such as retinal ganglion cells attributed to interleukin-1 and as therapeutic agents for ophthalmopathy such as glaucoma or retinal diseases exemplified by occlusion of retinal vessels, diabetic retinopathy, ischemic optic neuropathy, macular degeneration, retinitis pigmentosa, Leber's disease and the like.

### Industrial Applicability

The present invention provides protectants for optic nerve cells such as retinal ganglion cells and therapeutic agents for ophthalmopathy such as glaucoma or retinal diseases comprising compounds having inhibitory activities of interleukin-1 as active ingredients.

## Claims

1. A protectant for optic nerve cells comprising a compound having an inhibitory activity of interleukin-1 as an active ingredient.

2. The protectant as claimed in claim 1, wherein the optic nerve cells are retinal ganglion cells.

3. A therapeutic agent for ophthalmopathy comprising a compound having an inhibitory activity of interleukin-1 as an active ingredient.

4. The therapeutic agent as claimed in claim 3, wherein the ophthalmopathy is glaucoma.

5. The therapeutic agent as claimed in claim 3, wherein the ophthalmopathy is a retinal disease.

6. The therapeutic agent as claimed in claim 5, wherein the retinal disease is occlusion of retinal vessels, diabetic retinopathy, ischemic optic neuropathy, macular degeneration, retinitis pigmentosa or Leber's disease.

7. A method of protecting optic nerve cells comprising administering to a patient an effective amount of a composition comprising a compound having an inhibitory activity of interleukin-1 and a pharmaceutically acceptable additive.

8. The method as claimed in claim 7, wherein the optic nerve cells are retinal ganglion cells.

9. A method of treating ophthalmopathy comprising administering to a patient an effective amount of a composition comprising a compound having an inhibitory activity of interleukin-1 and a pharmaceutically acceptable additive.

10. The method as claimed in claim 9, wherein the ophthalmopathy is glaucoma.

11. The method as claimed in claim 9, wherein the ophthalmopathy is a retinal disease.

12. The method as claimed in claim 11, wherein the retinal disease is occlusion of retinal vessels, diabetic retinopathy, ischemic optic neuropathy, macular degeneration, retinitis pigmentosa or Leber's disease.

13. Use of a compound having an inhibitory activity of interleukin-1 in the manufacture of a protectant for optic nerve cells.

14. The use as claimed in claim 13, wherein the optic nerve cells are retinal ganglion cells.

15. Use of a compound having an inhibitory activity of interleukin-1 in the manufacture of a therapeutic agent for ophthalmopathy.

16. The use as claimed in claim 15, wherein the ophthalmopathy is glaucoma.

17. The use as claimed in claim 15, wherein the ophthalmopathy is a retinal disease.

18. The use as claimed in claim 17, wherein the retinal disease is occlusion of retinal vessels, diabetic retinopathy, ischemic optic neuropathy, macular degeneration, retinitis pigmentosa or Leber's disease.
